# EUROPEAN PATENT SPECIFICATION

(11) **EP 1 055 430 B1**
(45) Date of publication and mention of the grant of the patent: **01.09.2010**
(21) Application number: 00110278.9
(22) Date of filing: 22.05.2000
(51) Int. Cl.: A61L 9/03, A01M 1/20

(54) **Electric vaporizer for deodorants and insecticidal liquids**
Elektrischer Zerstäuber für Deodorantien und flüssige Insektizide
Vaporisateur électrique pour déodorants et insecticides liquides

(30) Priority: 27.05.1999 IT BO990292
(43) Date of publication of application: 29.11.2000
(73) Proprietor: Eurvest S.A., 1420 Braine l'Alleud (BE)
(72) Inventor: Falchieri, Roberto, 40121 Bologna (IT); Lupotto, Paolo, 13020 Pila (IT)
(74) Representative: Hirsch & Associés

(56) References cited:
- EP-A- 0 249 926
- EP-A- 0 689 766
- GB-A- 1 589 264
- GB-A- 2 155 210
- DATABASE WPI Section Ch, Week 199634 Derwent Publications Ltd., London, GB; Class C07, AN 1996-336893 XP002147857 & JP 08 155018 A (CASIO COMPUTER CO LTD), 18 June 1996 (1996-06-18)
- PATENT ABSTRACTS OF JAPAN vol. 1997, no. 12, 25 December 1997 (1997-12-25) & JP 09 201155 A (DIA GOMME KK), 5 August 1997 (1997-08-05)

## Description

The present invention relates to an electric vaporizer, particularly for deodorant and insecticidal liquids and the like, of the type as disclosed in EP-A-0 689 766.

It is known that several types of electric vaporizers are currently commercially available being designed to introduce in the air a preset amount of liquid by evaporation produced by heating, in order to deodorize enclosed spaces or for insecticidal use.

These vaporizers are composed, in their simplest embodiment, by a heater surrounding a wick which draws from a container liquid to be evaporated. The embodiment is completed by an enclosure which supports the container and optionally by a switch and by a luminous indicator which indicates the operating condition.

In view of the requirement to adjust the amount of released liquid, which has arisen mainly but not exclusively in the fragrance market, some vaporizers are equipped with adjustment means.

Some commercially available vaporizers achieve said adjustment mechanically, by positioning the heater coaxially around the wick by means of a screw-operated vertical actuator.

Other vaporizers instead achieve the adjustment mechanically by turning the heater about an axis which is perpendicular to the wick.

These vaporizers are often inaccurate, since their operation depends on factors such as the mechanical position of the wick with respect to the heating element; this in fact causes an asymmetrical heating of the wick, consequently producing temperature gradients which, by saturating the pores of the wick, cause a loss of effectiveness in the electric vaporization of the liquid or of its active principle.

Another drawback of which currently commercially available vaporizers suffer is that the switch for switching the vaporizer on and off is constituted by mechanical parts which are added to the device that regulates the power dissipated in the heater.

GB-A-2080111 discloses an atmospheric vaporiser that includes a fluid receptacle surrounded by an electrical heating element, an electric on/off switch, an interval timer clock such that the vaporiser operates automatically by switching on and off at times set on the interval timer clock, and a temperature sensor arranged against the bottom of the fluid receptacle to ensure that the temperature of the receiver does not exceed a preset value. In another embodiment, the fluid receiver is controlled at a predetermined temperature to subject the receptacle to continuous heat at the predetermined temperature to vaporize the receptacle's contents continuously into the atmosphere.

GB-A-2155210 discloses a power supply circuit for an atmospheric vaporiser with an electric heater, having means for controlling the supply of electric current to the electric heater including an on/off time switch and a thermostat for controlling the supply of electric current to the heater in dependence of the temperature sensed in the vicinity of the reagent-containing container provided with an override switch for enabling electric current to be supplied to the heater when the internal time is in its off state and the temperature responsive means is in a condition to allow supply of electrical current to the heater.

The aim of the present invention is to eliminate the drawbacks noted above in conventional vaporizers, by providing an electric vaporizer, particularly for deodorant and insecticidal liquids and the like, in which it is possible to regulate the power dissipated in the heater.

Within the scope of this aim, an object of the present invention is to provide a vaporizer in which it is possible to limit the minimum operating temperature precisely, safeguarding its functionality which may otherwise cause blockage of the capillary wick due to the thickened liquid.

Another object of the present invention is to provide a switch which is inherent in the power regulator, differently from what is currently commercially available, in which the switch is separate.

Another object of the present invention is to provide a vaporizer which has a simple structure, is relatively easy to provide in practice, is safe in use, effective in operation, and relatively low in cost.

In accordance with the invention, there is provided an electric vaporizer, particularly for deodorant and insecticidal liquids and the like, as defined in the appended claims.

Further characteristics and advantages will become better apparent from a detailed description of preferred but not exclusive embodiments of an electric vaporizer, particularly for deodorant and insecticidal liquids and the like, according to the invention, illustrated only by way of non-limitative example in the accompanying drawings, wherein:

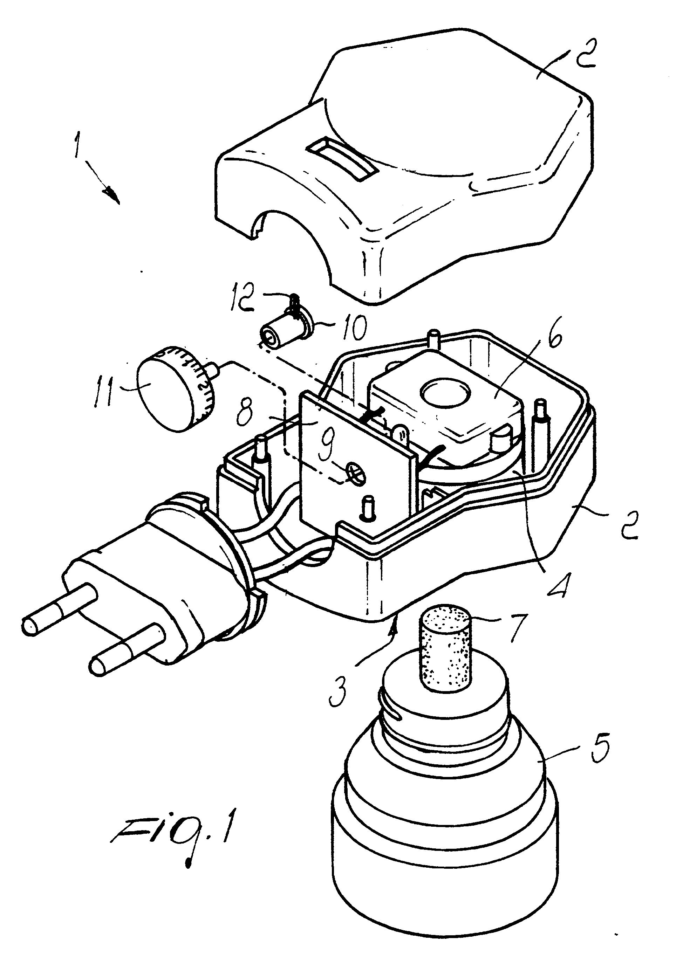

Figure 1 is an exploded perspective view of the electric vaporizer according to the invention;
Figure 2 is an electrical diagram of the electric vaporizer;
Figure 3 is a view of a second embodiment of the electrical diagram;
Figure 4 is a view of a third embodiment of the electrical diagram;
Figure 5 is a view of a fourth embodiment of the electrical diagram;
Figure 6 is a view of an embodiment of a resistor.

With reference to the above figures, 1 generally designates an electric vaporizer, particularly for deodorant and insecticidal liquids and the like, according to the invention. Said vaporizer comprises an enclosure 2 having, on a substantially flat face 3, a seat 4 which can be detachably engaged by a container 5 which accommodates a liquid to be evaporated.

The enclosure 2 internally accommodates a heater 6 which is supplied at low voltage by the electric mains and coaxially engages a wick 7 protruding from the top of the container 5.

Moreover, the enclosure 2 internally accommodates an electronic board 8 which supports switching means for driving on/off the heater 6 and means for setting the on/off threshold in order to adjust the power dissipated in the heater 6.

The electronic board 8 has a hole 9 rotatably engaged by a pivot 10 which radially supports a resistance selector 12. Said pivot is rigidly coupled to a knob-switch 11 which can be operated from outside.

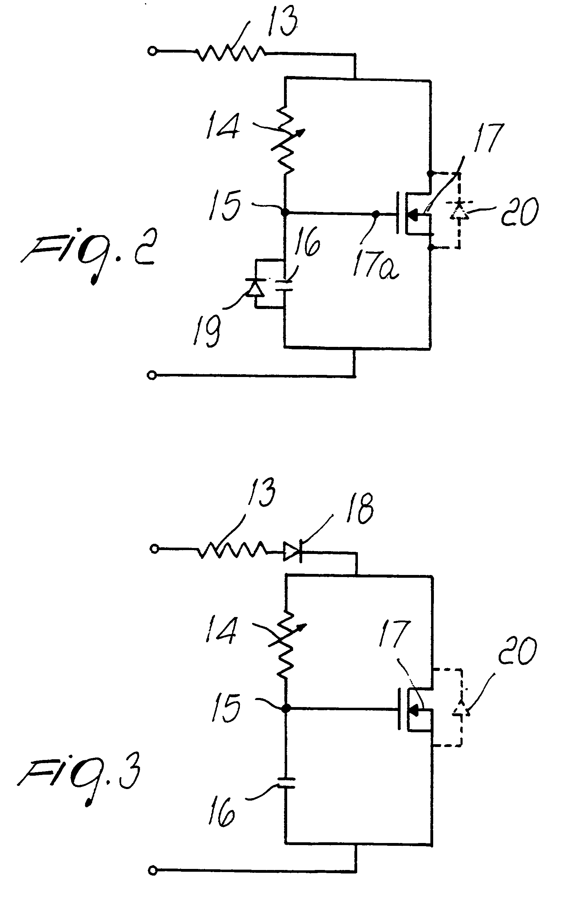

Figure 2 illustrates the electrical diagram of the vaporizer, in which the switching means for driving on/off the heater are in series to the heater 6, hereinafter schematically represented electrically by means of a resistor 13. The on/off switching means are arranged in parallel to the means for setting the on/off threshold and comprise a voltage divider, constituted by a variable resistor 14 which is connected in series on a common terminal 15 to a capacitor 16 which is connected in parallel to a first diode 19. The switching means are constituted by a MOSFET 17, which is connected by means of its gate terminal 17a to the common terminal 15, and by a second diode 20 which schematically represents the diode being intrinsic to the MOSFET.

In operation, the voltage divider is used to vary the voltage seen by the MOSFET, so that when said voltage exceeds the MOSFET switch-on threshold, said MOSFET starts to conduct, thus allowing the passage of current in the heater 6. In this manner, by varying the voltage seen by the MOSFET the power dissipated in the heater 6 is varied and therefore the level of liquid emitted by electronic adjustment between a medium value and a maximum value due to the presence of the diode which is intrinsic to the MOSFET. This embodiment in fact allows to perform adjustments only in the first half of the power supply period, while in the second half of the power supply period the first diode 19, by shorting the gate-source voltage, forces the passage of current for all the second half of the period through the second diode 20. In this manner, the power sent to the heater 6 is regulated electronically between a medium value and a maximum value. The present circuit is appropriate for the principle of electric vaporization, in which it is necessary to ensure a minimum evaporation temperature below which one must not go, because the vaporizer suddenly loses its effectiveness.

Figure 3 illustrates a second embodiment of the electric circuit shown in Figure 2.

Said second embodiment differs from the preceding one due to the absence of the diode 19 and due to the presence of a diode 18 in series to the heater 6. Said diode 18 causes the transmission of the power sent to the heater 6 to be unipolar, allowing power regulation between a null value and a maximum value.

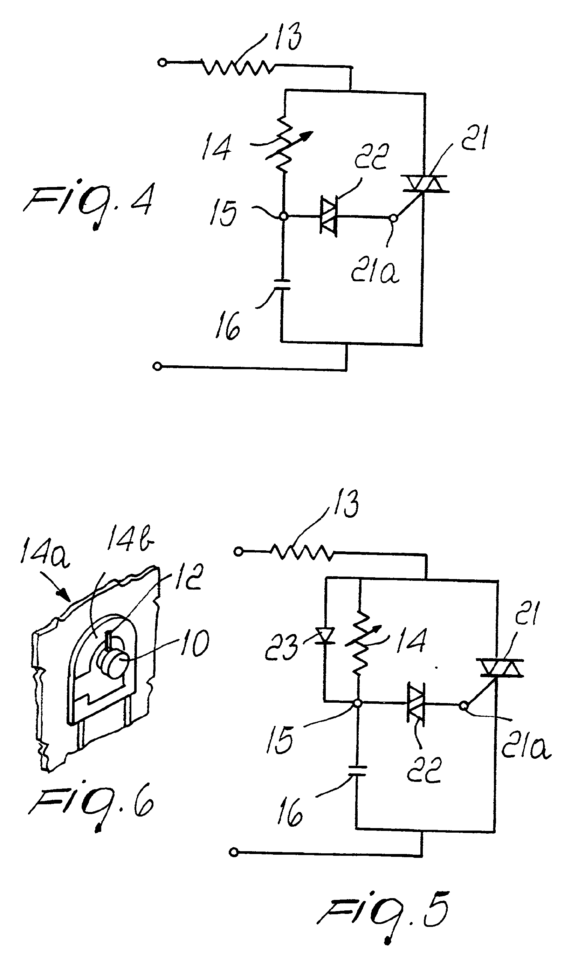

Figure 4 schematically shows the electric circuit according to a third embodiment, in which the switching means for driving the heater on/off comprise a triac 21 in which the gate terminal 21a is connected, by means of a diac 22, to the common terminal 15.

During operation, the voltage divider varies the phase of the signal seen by the diac 22. This allows to adjust the starting angle within the half-period of the supply frequency, and therefore allows to electronically regulate the on period of the triac 21, i.e., the time for which power is transmitted to the heater 6.

Figure 5 illustrates a fourth embodiment of the electric circuit shown in Figure 4.

The circuit differs from the preceding one due to the presence of a diode 23 in parallel to the variable resistor 14. This allows to regulate the power between a medium value and a maximum value, as indeed required by some electric vaporizers. This circuit in fact is quite appropriate to comply with the principle of electric vaporization, in which it is necessary to ensure a minimum evaporation temperature, below which one must not go because the vaporizer suddenly loses effectiveness.

The variable resistor 14 provided in the invention and in its different embodiments can be modified by continuous values or by discrete values. Said resistor is provided, for example, by way of a layer of carbon which is integrated on the board with a linear or semicircular arrangement and over which the resistance selector 12 passes, or as a separate component with respect to the electronic board 8.

With reference to a semicircular arrangement, the numeral 14a generally designates a variable resistor which can be integrated on the electronic board (see Figure 6). Said resistor, differently from commercially available components, has a layer of carbon 14b which can be limited to a value of a mechanical angle which reaches approximately 230° so that at said value the circuit is practically open due to the high value of the resistance. The variable resistor thus provided allows to spare the switch, since it directly performs this function, as in practice it eliminates the value of the current.

From the above description it is evident that the invention achieves the intended aim and objects, and in particular it is stressed that the adjustment of the power dissipated on the heater is achieved electronically and without moving mechanical parts.

Another very important aspect is that it is possible to provide a lower limit to the power dissipated on the heater, so that the minimum operating temperature does not drop below a certain minimum value, safeguarding the functionality of the vaporizer, which otherwise may cause the wick to block due to the thickened liquid.

Another very interesting aspect is due to the fact that the vaporizer intrinsically implements the switch function.

The electronic adjustment allows to provide the heater with the necessary power, thus optimizing energy consumption problems.

Moreover, electronic adjustment allows to make the heater independent of any interaction with mechanically moving parts, allowing an aesthetically optimized shape.

The invention thus conceived is susceptible of numerous modifications and variations, all of which are within the scope of the appended claims.

All the details may furthermore be replaced with other technically equivalent ones.

In practice, the materials employed, as well as the shapes and the dimensions, may be any according to requirements without thereby abandoning the protective scope of the appended claims.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An electric vaporizer, particularly for deodorant and insecticidal liquids and the like, comprising an enclosure (2) which accommodates a heater (6) which is supplied by the electric mains and is adapted to engage a wick (7) designed to be immersed in a container (5) which contains a liquid to be evaporated, **characterized in that** it comprises switching means (17) for driving said heater (6) on/off and means for setting (14) the on/off threshold of said switching means (17) in order to regulate the power dissipated in said heater (6) wherein said switching means (17) are connected in series to said heater (6) and said means for setting the on/off threshold comprise a voltage divider (14) which is connected in parallel to said switching means, said voltage divider comprising a variable resistor (14) and a capacitor (16) which are connected in series on a common terminal (15).

2. The electric vaporizer according to claim 1, **characterized in that** said switching means comprise a MOSFET (17) whose gate terminal (17a) is connected to said common terminal (15).

3. The electric vaporizer according to claim 2, **characterized in that** it comprises a first diode (19) which is connected in parallel to said capacitor (16) in order to regulate the power dissipated in said heater (6,13) between a medium value and a maximum value.

4. The electric vaporizer according to claim 2, **characterized in that** it comprises a diode (18) which is arranged in series between said heater (6,13) and said MOSFET (17) for the regulation of the power of said heater (6,13) between a null value and a maximum value.

5. The electric vaporizer according to claim 1, **characterized in that** said switching means comprise a triac (21) with a gate terminal (21a) which is connected to a diac (22) which is connected to said common terminal (15).

6. The electric vaporizer according to claim 5, **characterized in that** it comprises a diode (23) which is connected in parallel to said variable resistor (14) in order to regulate the power dissipated in said heater (6,13) between a medium value and a maximum value.

7. The electric vaporizer according to one or more of the preceding claims, **characterized in that** said variable resistor (14) can be modified by continuous or discrete values.

8. The electric vaporizer according to one or more of the preceding claims, **characterized in that** said variable resistor (14) acts as on/off switch.

## Patentansprüche

1. Elektrischer Zerstäuber, insbesondere für Deodorants und insektizide Flüssigkeiten und dergleichen, umfassend ein Gehäuse (2), das eine Heizvorrichtung (6) aufnimmt, die durch das Stromnetz versorgt wird und dazu geeignet ist, einen Docht (7) einzuschalten, der dazu gestaltet ist, in einen Behälter (5) eingetaucht zu werden, welcher eine zu verdampfende Flüssigkeit enthält, **dadurch gekennzeichnet, dass** er ein Schaltmittel (17) zum Ein- und Ausschalten der Heizvorrichtung (6) und ein Mittel zum Einstellen (14) der Ein/Aus-Schwelle des Schaltmittels (17), um die in der Heizvorrichtung (6) verbrauchte Leistung zu regulieren, umfasst, wobei das Schaltmittel (17) in Reihe mit der Heizvorrichtung (6) verbunden ist und das Mittel zum Einstellen der Ein/Aus-Schwelle einen Spannungsteiler (14) umfasst, der in Parallelschaltung mit dem Schaltmittel verbunden ist, wobei der Spannungsteiler einen Regelwiderstand (14) und einen Kondensator (16) umfasst, die in Reihe an eine gemeinsame Klemme (15) angeschlossen sind.

2. Elektrischer Zerstäuber nach Anspruch 1, **dadurch gekennzeichnet, dass** das Schaltmittel einen MOSFET (17) umfasst, dessen Gate-Klemme (17a) mit der gemeinsamen Klemme (15) verbunden ist.

3. Elektrischer Zerstäuber nach Anspruch 2, **dadurch gekennzeichnet, dass** er eine erste Diode (19) umfasst, die in Parallelschaltung mit dem Kondensator (16) verbunden ist, um die in der Heizvorrichtung (6, 13) verbrauchte Leistung zwischen einem mittleren Wert und einem Höchstwert zu regulieren.

4. Elektrischer Zerstäuber nach Anspruch 2, **dadurch gekennzeichnet, dass** er eine Diode (18) umfasst, die zur Regulierung der Leistung der Heizvorrichtung (6, 13) zwischen einem Null-Wert und einem Höchstwert in Reihe zwischen der Heizvorrichtung (6, 13) und dem MOSFET (17) angeordnet ist.

5. Elektrischer Zerstäuber nach Anspruch 1, **dadurch gekennzeichnet, dass** das Schaltmittel einen Triac (21) mit einer Gateklemme (21a), die mit einem Diac (22) verbunden ist, der mit der gemeinsamen Klemme (15) verbunden ist, umfasst.

6. Elektrischer Zerstäuber nach Anspruch 5, **dadurch gekennzeichnet, dass** er eine Diode (23) umfasst, die in Parallelschaltung mit dem Regelwiderstand (14) verbunden ist, um die in der Heizvorrichtung (6, 13) verbrauchte Leistung zwischen einem mittleren Wert und einem Höchstwert zu regulieren.

7. Elektrischer Zerstäuber nach einem oder mehreren der vorhergehenden Ansprüche, **dadurch gekennzeichnet, dass** der Regelwiderstand (14) durch fortlaufende oder diskrete Werte abgeändert werden kann.

8. Elektrischer Zerstäuber nach einem oder mehreren der vorhergehenden Ansprüche, **dadurch gekennzeichnet, dass** der Regelwiderstand (14) als Ein/Ausschalter wirkt.

## Revendications

1. Diffuseur électrique, en particulier pour des liquides désodorisants et insecticides, et similaires, comprenant une enceinte (2) logeant un élément chauffant (6) alimenté sur secteur et qui est adapté pour venir en prise avec une mèche (7) conçue pour être immergée dans un récipient (5) qui contient un liquide destiné à être évaporé, **caractérisé en ce qu'**il comprend des moyen de commutation (17) pour mettre ledit élément chauffant (6) en marche/arrêt et des moyens pour régler (14) le seuil de marche/arrêt desdits moyens de commutation (17) afin de réguler la puissance dissipée dans ledit élément chauffant (6), lesdits moyens de commutation (17) étant reliés en série avec ledit élément chauffant (6) et lesdits moyens pour régler le seuil de marche/arrêt comprenant un diviseur de tension (14), qui est relié en parallèle avec lesdits moyens de commutation, ledit diviseur de tension comprenant une résistance variable (14) et un condensateur (16) reliés en série sur une borne commune (15).

2. Le diffuseur électrique selon la revendication 1, **caractérisé en ce que** lesdits moyens de commutation comprennent un transistor MOSFET (17) dont la borne de grille (17a) est reliée à ladite borne commune (15).

3. Le diffuseur électrique selon la revendication 2, **caractérisé en ce qu'**il comprend une première diode (19) qui est reliée en parallèle avec ledit condensateur (16) afin de réguler la puissance dissipée dans ledit élément chauffant (6,13) entre une valeur moyenne et une valeur maximale.

4. Le diffuseur électrique selon la revendication 2, **caractérisé en ce qu'**il comprend une diode (18) agencée en série entre ledit élément chauffant (6,13) et ledit transistor MOSFET (17) pour la régulation de la puissance dudit élément chauffant (6,13) entre une valeur nulle et une valeur maximale.

5. Le diffuseur électrique selon la revendication 1, **caractérisé en ce que** lesdits moyens de commutation comprennent un triac avec une borne de grille (21a) reliée à un diac (22) qui est relié à ladite borne commune (15).

6. Le diffuseur électrique selon la revendication 5, **caractérisé en ce qu'**il comprend une diode (23) qui est branchée en parallèle avec ladite résistance variable (14) afin de réguler la puissance dissipée dans ledit élément chauffant (6,13) entre une valeur moyenne et une valeur maximale.

7. Le diffuseur électrique selon l'une quelconque des revendications précédentes, **caractérisé en ce que** ladite résistance variable (14) peut être modifiée de façon continue ou selon des valeurs discrètes.

8. Le diffuseur électrique selon l'une quelconque des revendications précédentes, **caractérisé en ce que** ladite résistance variable (14) fonction comme un commutateur de marche/arrêt.
